**Europäisches Patentamt**

**European Patent Office**

**Office européen des brevets**

(19)

(11) Veröffentlichungsnummer : **0 022 064**
**B1**

(12) **EUROPÄISCHE PATENTSCHRIFT**

(45) Veröffentlichungstag der Patentschrift :
24.08.83

(51) Int. Cl.³ : **A 61 L 15/01**, A 61 L 15/03//
A61K31/765

(21) Anmeldenummer : 80810178.6

(22) Anmeldetag : 28.05.80

(54) **Polyvinylalkohole zur Behandlung von Wunden.**

(30) Priorität : 31.05.79 CH 5089/79

(43) Veröffentlichungstag der Anmeldung :
07.01.81 Patentblatt 81/01

(45) Bekanntmachung des Hinweises auf die Patenterteilung : 24.08.83 Patentblatt 83/34

(84) Benannte Vertragsstaaten :
BE CH DE FR GB IT LI NL SE

(56) Entgegenhaltungen :
DE A 2 424 506
FR A 1 505 318
FR A 2 215 230
GB A 696 608
US A 2 693 438

N.M. BIKALES : « Encyclopedia of Polymer Science and Technology », Band 14, 1974, John Wiley, New York, U.S.A. *Seiten 156, 157, 162, 163*

(73) Patentinhaber : Zyma SA
Route de l'Etraz
CH-1260 Nyon (CH)

(72) Erfinder : Mirkovitch, Velimir, Dr.
Avenue de Lavaux 78/B
CH-1009 Pully (CH)

(74) Vertreter : Meyer, Hans Rudolf et al
Patentabteilung der CIBA-GEIGY AG Postfach
CH-4002 Basel (CH)

Jouve, 18, rue St-Denis, 75001 Paris, France

Polyvinylalkohole zur Behandlung von Wunden

Gegenstand der Erfindung ist die Verwendung eines in kaltem Wasser unlöslichen Polyvinylalkohols, in Pulver- oder Granulatform, zur Behandlung von Wunden.

Es ist bekannt, dass die Schliessung von offenen Wunden jeder Art durch Behandlung mit trocknenden und reinigenden Mitteln beschleunigt werden kann. So werden gemäss FR-A 2 215 230 zur Behandlung von verletzten und Flüssigkeit abscheidenden Körperoberflächen wasserunlösliche hydrophile vernetzte Polymere verwendet.

Ebenfalls bekannt ist, dass Gele und Filme von Polyvinylalkoholen verschiedenen Molekulargewichts in Form von Wundbandagen als Träger für Heilmittel verwendet werden.

Es wurde nun gefunden, dass gewisse in kaltem Wasser unlösliche Polyvinylalkohole, in Pulver- oder Granulatform, selbst eine wundheilende Wirksamkeit besitzen. Die vorliegende Erfindung betrifft demnach die in kaltem Wasser unlöslichen und in kochendem Wasser weniger als 12 % bis 13 % löslichen Polyvinylalkohole mit einem 99 % übersteigenden Hydrolysegrad in Pulver- oder Granulatform als Heilmittel zur Wundbehandlung.

Die erfindungsgemäss zu verwendenden Polyvinylalkohole sind fest und haben eine grosse Fliessfähigkeit ; sie sind hygroskopisch und in kaltem Wasser (Temperatur kleiner als 20 °C, ja bis gegen 50 °C), unlöslich, während ihre Löslichkeit in kochendem Wasser kleiner als 12 % bis 13 % ist, ihr Hydrolysegrad 99 % übersteigt. Solche Polyvinylalkohle sind z. B. unter dem Handelsnamen « Elvanol » Typ 71-30 ; HV ; 90-50 oder 85-60 käuflich.

Diese Polyvinylalkohole werden in Pulver- oder Granulatform verwendet, besonders in Granulatform, deren Einzelteile einen mittleren Durchmesser von 0,05 bis 0,4 mm, in erster Linie von 0,1 bis 0,2 mm aufweisen. Bevorzugt sind solche, deren Einzelteile einen mittleren Durchmesser von 0,15 mm haben.

Diese Polyvinylalkohole haben eine schwach desinfizierende Wirksamkeit. Um diese zu verstärken, kann man zur erfindungsgemässen Verwendung Desinfektionsmittel, wie sie z. B. zur Desinfektion der Haut oder von Wunden verwendet werden, oder Antibiotika, z. B. Neomycin oder Merfen (Handelsmarke = Phenylmercuriborat) beifügen.

Als Wunden, die in der Human- oder Veterinärmedizin erfindungsgemäss mit diesen Polyvinylalkoholen behandelt werden können, seien beispielsweise genannt die varikösen Geschwüre, Verbrennungen, besonders vom 2. und 3. Grad, traumatisch bedingte Wunden, besonders wenn sie infiziert sind, frische oder infizierte postoperative Wunden, der Decubitus, infizierte Haut (beispielsweise bei Pyodermie und/oder Mykosen) und Ekzeme.

Die Methode zur Wundbehandlung mit diesen in kaltem Wasser unlöslichen Polyvinylalkoholen besteht darin, dass man eine Wunde mit den pulver- oder granulatförmigen Polyvinylalkoholen überdeckt, nach einer gewissen Zeit, vorzugsweise nach 12 bis 24 Stunden, die entstandene Kruste entfernt und erneut die so blossgelegte Wunde mit den Polyvinylalkoholen überdeckt ; wobei diese Behandlung mindestens während 2 bis 3 Tagen wiederholt wird. Durch diese Behandlung wird die Wunde getrocknet und gereinigt und die Abheilung der Wunde erfolgt merkbar schneller als bei entsprechenden mit bekannten Mitteln behandelten oder unbehandelten Wunden. So konnte bei Ratten (weiblich, Wistar) wie auch bei Hunden (Mischling), die experimentell verwundet wurden, und dann in der beschriebenen Weise behandelt wurden, indem man die Wunde mit den genannten Polyvinylalkoholen überdeckt, die entstandene Kruste nach einem Tag entfernt, die Wunde erneut mit den genannten Polyvinylalkoholen überdeckt und die entstandene Kruste nach einem Tag entfernt und die Behandlung nochmals wiederholt, festgestellt werden, dass bereits nach 3 Tagen die Wundheilung weit fortgeschritten ist und dass im Vergleich mit entsprechenden, unbehandelten Wunden, die vollkommene Wundheilung mindestens zwei Tage schneller erfolgt.

Auch klinische Resultate zeigen, dass Wunden verschiedener Art günstig auf diese Behandlung ansprechen. So war bei einem 60-jährigen Patienten, dem ein Bein amputiert werden musste und dessen Wunde während 3 Monaten gegenüber verschiedensten anderen Behandlungen refraktär war, bereits nach 3-tägiger Behandlung mit den genannten Polyvinylalkoholen die Wunde geschlossen und der Patient konnte beginnen, eine Prothese zu tragen. In einem anderen Fall führte bei einer 80-jährigen Patientin, die während 20 Jahren unter einem varikösen Geschwür litt, die oben genannte Behandlung bereits nach 2 Wochen zur Abheilung des Geschwürs.

**Ansprüche**

1. Die in kaltem Wasser unlöslichen und in kochendem Wasser weniger als 12 % bis 13 % löslichen Polyvinylalkohole mit einem 99 % übersteigenden Hydrolysegrad, in Pulver- oder Granulatform als Heilmittel zur Wundbehandlung, gegebenenfalls zusammen mit Desinfektionsmitteln und/oder Antibiotika.

2. Pharmazeutisches Präparat zur Wundbehandlung mit einem Gehalt an einem in kaltem Wasser unlöslichen und in kochendem Wasser weniger als 12 % bis 13 % löslichen Polyvinylalkohol mit einem 99 % übersteigenden Hydrolysegrad in Pulver- oder Granulatform.

3. Pharmazeutisches Präparat gemäss Patentanspruch 1, dadurch gekennzeichnet, dass die Einzelteilchen des Pulvers oder Granulats einen mittleren Durchmesser von 0,05 bis 0,4 mm besitzen.

4. Pharmazeutisches Präparat gemäss Patentanspruch 1, dadurch gekennzeichnet, dass die Einzelteilchen des Pulvers oder Granulates einen mittleren Durchmesser von 0,1 bis 0,2 mm besitzen.

5. Pharmazeutisches Präparat gemäss einem der Patentansprüche 1 bis 3, dadurch gekennzeichnet, dass die Einzelteilchen des Granulats einen mittleren Durchmesser von 0,15 mm besitzen.

6. Pharmazeutisches Präparat gemäss einem der Patentansprüche 1 bis 4, dadurch gekennzeichnet, dass der Polyvinylalkohol noch Desinfectionsmittel und/oder Antibiotika enthält.

## Claims

1. Polyvinyl alcohols insoluble in cold water and soluble in boiling water to the extent of less than 12 to 13 % and with a degree of hydrolysis exceeding 99 %, in the form of powders or granulates, optionally together with disinfectants and/or antibiotics, as medicaments for the treatment of wounds.

2. A pharmaceutical preparation for the treatment of wounds, which preparation contains a polyvinyl alcohol insoluble in cold water and soluble in boiling water to the extent of less than 12 to 13 % and with a degree of hydrolysis exceeding 99 %, in the form of a powder or a granulate.

3. A pharmaceutical preparation according to Claim 1, characterised in that the individual particles of the powder or granulate have a mean diameter of 0.05 to 0.4 mm.

4. A pharmaceutical preparation according to Claim 1, characterised in that the individual particles of the powder or granulate have a mean diameter of 0.1 to 0.2 mm.

5. A pharmaceutical preparation according to any one of Claims 1 to 3, characterised in that the individual particles of the granulate have a mean diameter of 0.15 mm.

6. A pharmaceutical preparation according to any one of Claims 1 to 4, characterised in that the polyvinyl alcohol contains disinfectants and/or antibiotics.

## Revendications

1. Alcools polyvinyliques insolubles dans l'eau froide et solubles à moins de 12 à 13 % dans l'eau chaude avec un degré d'hydrolyse dépassant 99 %, sous forme de poudre ou de granulé comme médicament pour le traitement des blessures, éventuellement avec des désinfectants et/ou antibiotiques.

2. Préparation pharmaceutique pour le traitement des blessures, contenant un alcool polyvinylique insoluble dans l'eau froide et soluble à moins de 12 à 13 % dans l'eau bouillante, avec un degré d'hydrolyse dépassant 99 %, sous forme de poudre ou de granulé.

3. Préparation pharmaceutique selon la revendication 1, caractérisée en ce que les particules isolées de la poudre ou du granulé présentent un diamètre moyen de 0,05 à 0,4 mm.

4. Préparation pharmaceutique selon la revendication 1, caractérisée en ce que les particules isolées de la poudre ou du granulé présentent un diamètre moyen de 0,1 à 0,2 mm.

5. Préparation pharmaceutique selon l'une des revendications 1 à 3, caractérisée en ce que les particules isolées du granulé présentent un diamètre moyen de 0,15 mm.

6. Préparation pharmaceutique selon l'une des revendications 1 à 4, caractérisée en ce que l'alcool polyvinylique contient encore des désinfectants et/ou antibiotiques.